# EUROPEAN PATENT APPLICATION

(11) **EP 3 960 168 A1**
(43) Date of publication of application: **02.03.2022**
(21) Application number: 20193422.1
(22) Date of filing: 28.08.2020
(51) Int. Cl.: A61K 31/198, A61K 31/53, A61P 35/00

(54) **COMPOSITIONS AND METHODS COMPRISING D-CYSTEINE OR A DERIVATIVE THEREOF**

(71) Applicant: Université de Genève, 1211 Genève (CH)
(72) Inventor: Zangari, Joséphine, 1211 Genève 4 (CH); Martinou, Jean-Claude, 1211 Genève 4 (CH)
(74) Representative: KATZAROV S.A.

(57) **Abstract**

The present disclosure provides compositions and methods for their use in the treatment and/or prevention of a ferrotoxic disease, an iron related disease or disorder, or a cancer, the compositions comprising a D-cysteine or a derivative thereof, an isomer or a salt of said D-cysteine or derivative.

## Description

### FIELD OF THE INVENTION

The present disclosure provides compositions and methods for their use in the treatment and/or prevention of a cancer, a ferrotoxic disease, or an iron related disease or disorder, the compositions comprising a D-cysteine or a derivative thereof, an isomer or a salt of said D-cysteine or derivative.

### BACKGROUND OF THE INVENTION

Iron is an essential metal not only for oxygen delivery but also for cellular processes, including ATP production through oxidative phosphorylation and DNA biosynthesis. Iron is also an important molecule involved in infection, inflammation, and cell death and several studies point to a key role of iron in cancer.

Cancer is a leading cause of death worldwide, accounting for approximately 10 million deaths in 2019. Lung cancer is the most frequently encountered cancer and its incidence continues to grow. The two main types of lung cancer are small-cell lung cancer (SCLC) and non-small-cell lung cancer (NSCLC). The latter is the most common form, accounting for more than 80 % of cases. Lung cancer has a poor prognosis, which is due in part to late diagnosis.

Malignant mesothelioma is an aggressive, fatal tumor of the pleura or peritoneum and is closely associated with long-term exposure to asbestos fibers^{1,2}. Malignant pleural mesothelioma (MPM) is the most common form with a long latency period (20-50 years) between asbestos exposure and tumor development, explaining why the incidence of mesothelioma continues to go up while the use of asbestos has been banned in the United States and many European countries^{3,4}. MPM has notoriously poor prognosis, and most patients die within two years after diagnosis due to limited efficacy of current treatment modalities⁵.

There is therefore an urgent need to develop effective cancer treatments, in particular treatments that do not impair proliferation of normal cells and are thus selective to cancer cells.

### BRIEF DESCRIPTION OF THE FIGURES

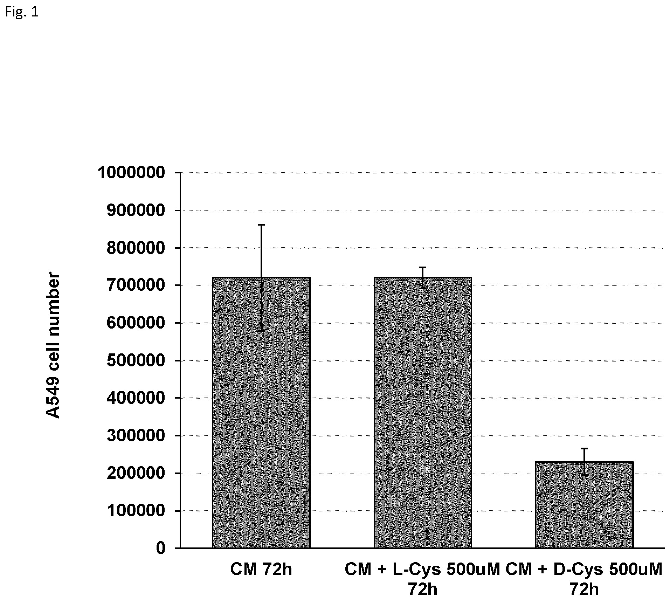

**Figure 1****.** Effect of L- and D-Cysteine, each tested at 500 microM, on the proliferation of A549 cells. Cells were counted 3 days after addition of L- or D-Cysteine. (CM= culture medium)

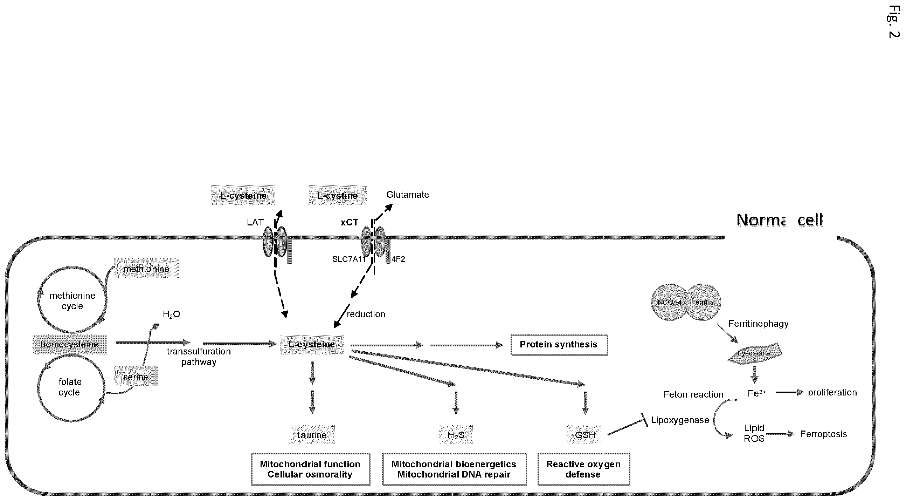

**Figure 2****.** The cellular pathway of synthesis and metabolism of cysteine

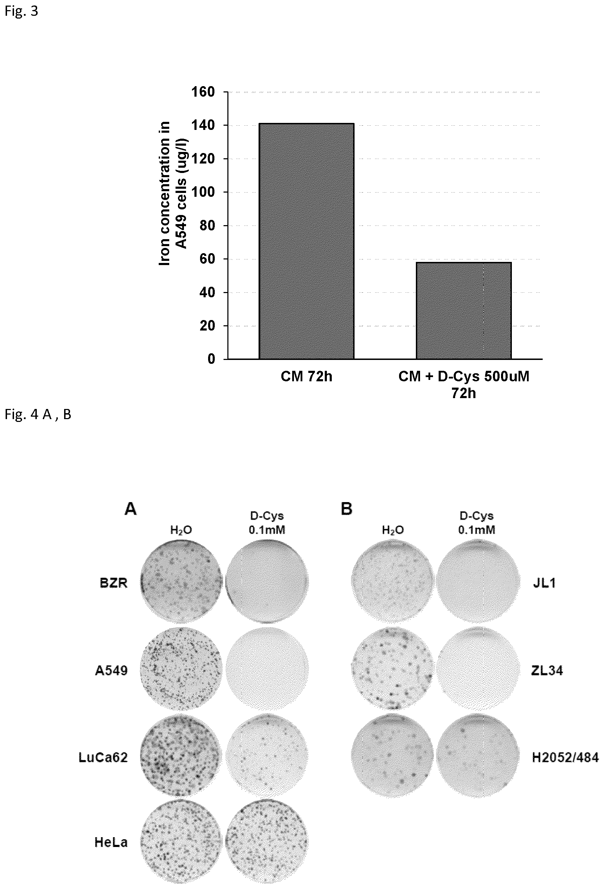

**Figure 3****.** A: Iron concentration in A549 cells cultured in the presence or absence of D-Cysteine for 72h. Iron was measured using ICP-MS (Inductively coupled plasma mass spectrometry).

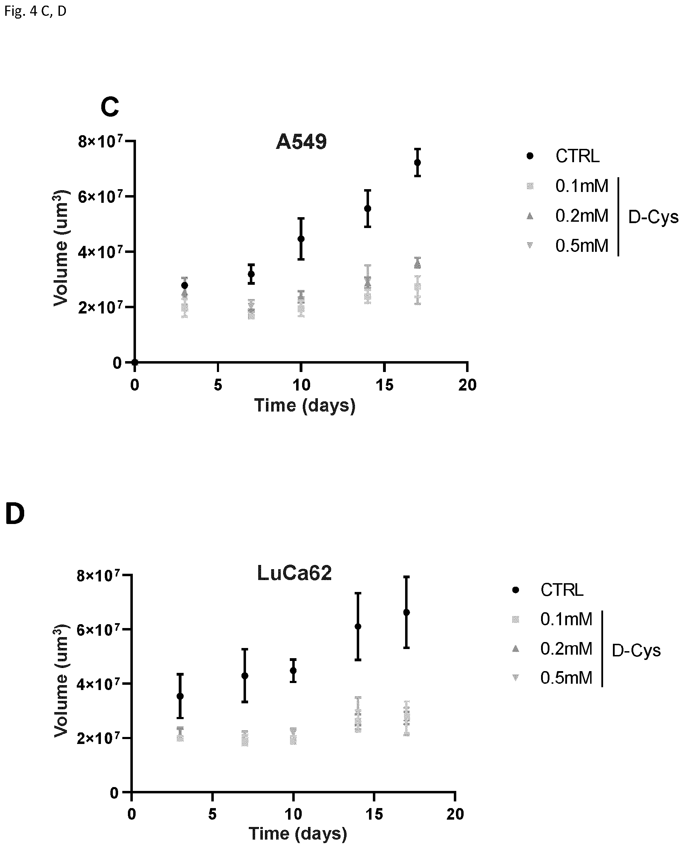

**Figure 4****: D-cysteine affects tumor cell viability.** A-B: Colony formation assays in presence or absence 0.1 mM D-cysteine (D-Cys) for 2 weeks. A: Lung tumor cell lines + cervix cell line (HeLa). B: MPM cell lines. C: A549 and (D) LuCa62 spheroid formation in presence or absence of 0.1mM, 0.2mM or 0.5 mM D-cysteine for 17 days.

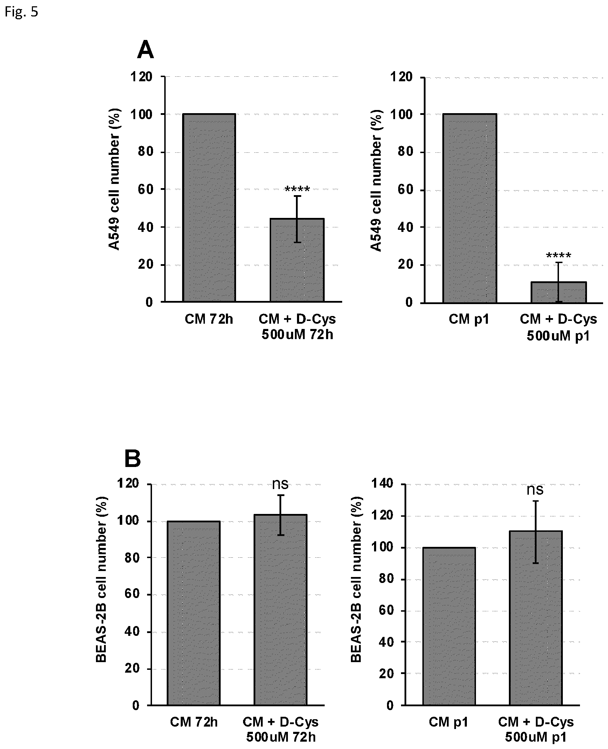

**Figure 5****: D-cysteine impairs proliferation of tumor cells but not normal cells.** A549 lung tumor cells (A) or BEAS-2B normal bronchial epithelial cells (B) were cultured in complete media (CM) in the absence or presence of 500 µM D-cysteine (D-Cys). Cell numbers were analyzed after 72 h and after splitting of cells (p1). Cell numbers are expressed as % of cells cultured in the absence of D-cysteine. **** P<0.0001.

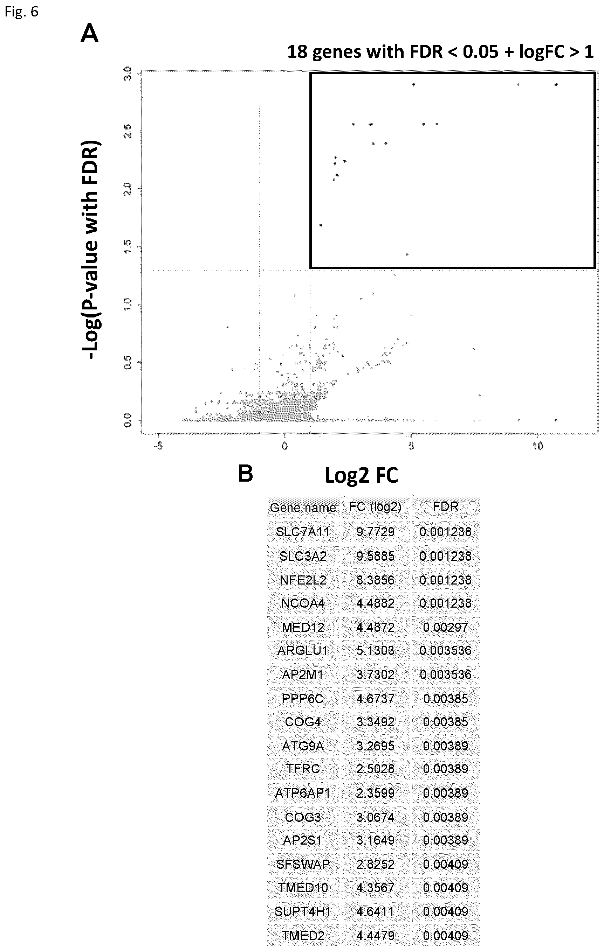

**Figure 6****: Candidate genes potentially involved in D-cysteine toxicity.** NGS analysis of D-cysteine-resistant cells following infection with the CRISPR library. Results of two genome-wide CRISPR-Cas9 KO screens were pooled. A. Volcano plot of D-Cysteine-treated vs ctrl cells (the most significant genes are in the top right rectangle). B. Identification of the 18 candidate genes most implicated in D-cysteine toxicity. FC=Fold change. FDR=False discovery rate.

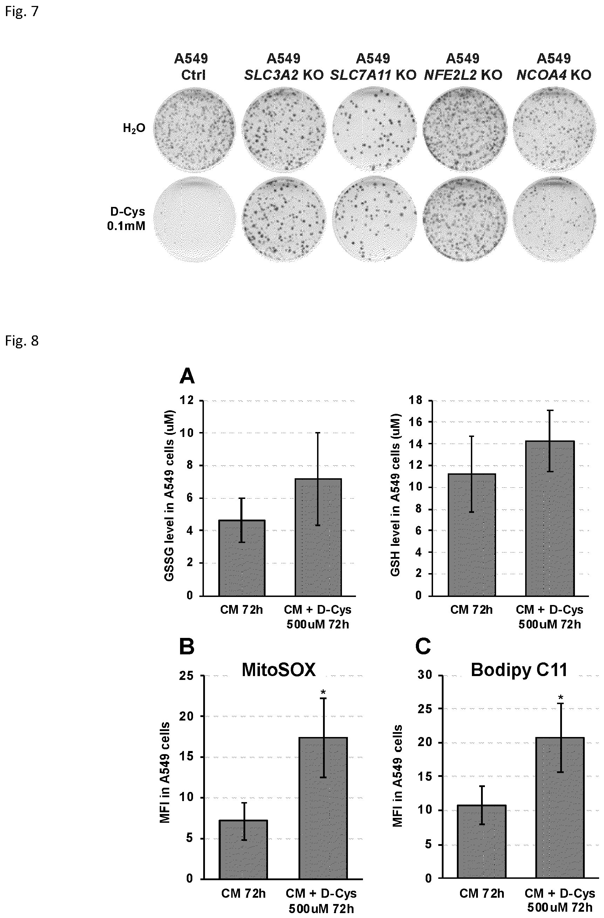

**Figure 7****: Validation of targets involved in D-cysteine toxicity.** Colony formation assays in which the cells were grown in the absence or presence of 0.1 mM D-cysteine (D-Cys) for 2 weeks.
**Figure 8****: D-cysteine induces an increase in mitochondrial ROS and lipid peroxidation in lung tumor cells.** A549 cells were cultured for 72 h in complete medium (CM) with or without 500 µM D-cysteine (D-Cys) and different cellular characteristics were analyzed. A: Oxidized (GSSG) and total (GSH) glutathione levels. B: Mitochondrial ROS production by MitoSOX staining. C: Lipid peroxidation by Bodipy C11 staining. MFI=Mean of Fluorescence Intensity (Flow cytometry). * P<0.05.

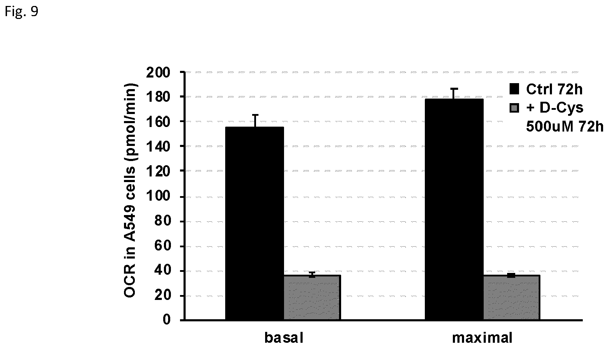

**Figure 9****: D-cysteine impairs mitochondrial respiration of lung tumor cells.** A549 cells were cultured for 72 h in complete media (CM) supplemented or not with 500 µM D-cysteine (D-Cys), and oxygen consumption rate (OCR) was quantified both before (basal respiration) and after sequential addition of oligomycin and FCCP (maximal respiration).

### SUMMARY OF THE INVENTION

The present invention provides a pharmaceutical composition comprising a D-cysteine or a derivative thereof, an isomer or a salt of said D-cysteine or derivative, for use in the treatment and/or prevention of a cancer expressing the cysteine/glutamate antiporter xCT.

Another aspect of the invention concerns a pharmaceutical composition comprising a D-cysteine or a derivative thereof, an isomer or a salt of said D-cysteine or derivative, for use in the treatment and/or prevention of a ferrotoxic disease or an iron related disease or disorder.

A further aspect of the invention concerns a pharmaceutical composition comprising a D-cysteine or a derivative thereof, an isomer or a salt of said D-cysteine or derivative, for use in the treatment and/or prevention of a cancer in a patient, wherein the method comprises: (i) determining whether the cancer from the patient expresses the cysteine/glutamate antiporter xCT; and (ii) if the cancer from the patient expresses said cysteine/glutamate antiporter xCT, administering to the patient an effective amount of the pharmaceutical composition.

### DESCRIPTION

Although methods and materials similar or equivalent to those described herein can be used in the practice or testing of the present invention, suitable methods and materials are described below. All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise/comprising" is generally used in the sense of include/including, that is to say permitting the presence of one or more features or components. The terms "comprise" and "comprising" also encompass the more restricted ones "consist" and "consisting", respectively.

As used in the specification and claims, the singular form "a", "an" and "the" include plural references unless the context clearly dictates otherwise.

As used herein, "at least one" means "one or more", "two or more", "three or more", etc.

As used herein the terms "subject"/"subject in need thereof", or "patient"/"patient in need thereof" are well-recognized in the art, and, are used interchangeably herein to refer to a mammal, including dog, cat, rat, mouse, monkey, cow, horse, goat, sheep, pig, camel, and, most preferably, a human. In some cases, the subject is a subject in need of treatment or a subject with a disease or disorder. However, in other aspects, the subject can be a normal subject. The term does not denote a particular age or sex. Thus, adult and newborn subjects, whether male or female, are intended to be covered.

In one aspect, the subject is a human, most preferably a human suffering from cancer, cancer metastasis or a human that might be at risk of suffering from cancer and/or cancer metastasis.

In another aspect, the subject is a human, most preferably a human suffering from a ferrotoxic disease or an iron related disease or disorder or a human that might be at risk of suffering from a ferrotoxic disease or an iron related disease or disorder.

While focusing on understanding the role of amino-acids in the pivotal role of pyruvate metabolism in carcinogenesis, the Inventors surprisingly discovered the role of the thiol group of cysteine in affecting the growth of cancer cells, in particular cancer cells expressing the cysteine/glutamate antiporter xCT. As demonstrated in the experimental examples, only cysteine, D- form, had potent selective inhibitory activities on cancer cells, in particular those expressing or overexpressing the cysteine/glutamate antiporter xCT. In clear contrast, the L form of cysteine has no adverse effect on the growth of cancer cells (Figure 1).

Cysteine contains a reactive thiol side chain that endows it with several specific properties. Cysteine can be synthesized *de novo* from serine and methionine in a pathway known as transsulfuration (Figure 2). While this pathway has been shown to contribute to cysteine production in cancer cells⁶, the majority of intracellular cysteine is taken up from the extracellular environment. The physiological concentration of L-cysteine in the blood is around 0.1-0.2 mM - either as cysteine⁷ or as its derivative cystine, which is an oxidized dimeric form of cysteine. While L-cysteine, like other L-amino acids, is imported into cells by the L-amino acid transporters (LAT)⁸, L-cystine is transported into cells via the cystine/glutamate antiporter xCT, which is composed of the *SLC7A11* transporter, selective for cystine, and a separate protein, known as 4F2 heavy chain or CD98, encoded by the *SLC3A2* gene, which localizes xCT to the cell membrane⁹. Importantly, both the *SLC3A2* and *SLCA11* genes are highly expressed in many cancer cells, in particular those that are p53 negative¹⁰. Once in the cytosol, cystine is reduced into cysteine by thioredoxin reductase 1 and glutathione reductase¹¹. In addition to its role in protein synthesis, cysteine is used to produce the amino acid taurine which plays a role in mitochondrial function and in the control of cellular osmolarity, the gasotransmitter hydrogen sulfide (H₂S), and iron-sulfur clusters required for respiration and the activity of several key enzymes¹².

One of the key functions of cysteine in cancer is its role in reactive oxygen defense as part of several antioxidant systems¹³. Indeed, cysteine has an important role in the synthesis of the antioxidant glutathione. The potential importance of glutathione metabolism in cancer is evidenced by the observation that glutathione is one of the most significantly increased metabolites in tumors relative to normal tissue^{14,15}.

Besides cysteine, many cancers accumulate large amounts of iron to support increased proliferation¹⁶. Interestingly, inhibition of cystine uptake induces a recently discovered form of cell death known as ferroptosis, an iron-dependent form of regulated cell death characterized by the accumulation of iron-based lipid reactive oxygen species^{17,18}. Cancer cells exhibit more dependence on iron and enhanced sensitivity to ferroptosis than normal cells¹⁹. Triggering ferroptosis is currently considered as a promising approach to treat cancer²⁰⁻²² and thus also considered in the present invention.

Ferritin represents the major form of stored iron via its iron-filled cage structure^{23,24}. In general, ferritin functions in regulating the balance between reduced (Fe²⁺) and oxidized (Fe³⁺) iron states. Fe²⁺ is incorporated into ferritin and is further oxidized to Fe³⁺ by FTH1, which leads to inert deposits of Fe³⁺ that are unavailable for intracellular use or the generation of reactive oxygen species (ROS)²⁵. For utilization, iron needs to be released from ferritin and reduced again to Fe²⁺. The release of iron from ferritin requires the lysosomal proteolytic activity²⁶. Autophagic degradation of ferritin represents a crucial mechanism to maintain iron homeostasis²⁷. In 2014, Mancias et al. reported the role of the nuclear receptor coactivator 4 (NCOA4) as a specific cargo receptor that mediates the autophagic degradation of ferritin, a phenomenon called "ferritinophagy", to release free iron. NCOA4-mediated ferritinophagy maintains intracellular iron homeostasis by facilitating ferritin iron storage or release according to cellular demand²⁸. Importantly, it has been shown that increased ferritinophagy is critical to initiate ferroptosis through promoting accumulation of ferric iron, which through the Fenton reaction leads to production of ROS and lipid peroxidation^{22,29}.

For years, D-amino acids were thought to have a minor function in biological processes compared to their L-enantiomers. Nevertheless, it was found that D-serine plays an important role in the brain, where it acts as a modulator of the glutamatergic neurotransmission³⁰. Moreover, it has been shown that the D-amino acid oxidase (DAAO), a flavoenzyme which oxidizes D-amino acids, has a pivotal role in motoneuron degeneration through D-serine regulation³¹.

On the other hand, D-cysteine was found to promote dendritic development *in vitro.* However, in contrast to D-serine, D-cysteine, like the D-enantiomeric form of the other amino acids, does not appear to be synthesized in animals.

The present invention thus provides compositions and pharmaceutical compositions comprising a D-cysteine a derivative thereof, or an isomer or a salt,of said D-cysteine or derivative. Preferably, these compositions comprise a therapeutically effective amount of D-cysteine or a derivative thereof, an isomer or a salt of said D-cysteine or derivative.

The term "therapeutically effective amount" as used herein means an amount of D-cysteine or a derivative thereof, an isomer or a salt of said D-cysteine or derivative, high enough to significantly positively modify the symptoms and/or condition to be treated, but low enough to avoid serious side effects (at a reasonable risk/benefit ratio), within the scope of sound medical judgment. The therapeutically effective amount of the D-cysteine or a derivative thereof, an isomer or a salt of said D-cysteine or derivative is selected in accordance with a variety of factors including type, species, age, weight, sex and medical condition of the patient; the severity of the condition to be treated; the route of administration; the renal and hepatic function of the patient.

In an aspect of the invention, the composition or pharmaceutical composition comprising a D-cysteine or a derivative thereof, an isomer or a salt of said D-cysteine or derivative is for use in the treatment and/or prevention of a cancer, preferably a cancer expressing the cysteine/glutamate antiporter xCT. In certain aspects, the cancer expressing the cysteine/glutamate antiporter xCT is p53 negative.

"Treatment" or "treating" is an approach for obtaining beneficial or desired results including clinical results. Beneficial or desired clinical results may include one or more of the following: a) inhibiting the disease or condition, (e.g., decreasing one or more symptoms resulting from the disease or condition, and/or diminishing the extent of the disease or condition); b) slowing or arresting the development of one or more clinical symptoms associated with the disease or condition (e.g., stabilizing the disease or condition, preventing or delaying the worsening or progression of the disease or condition, and/or preventing or delaying the spread (e.g., metastasis) of the disease or condition); and/or c) relieving the disease, that is, causing the regression of clinical symptoms (e.g., ameliorating the disease state, providing partial or total remission of the disease or condition, enhancing effect of another medication, delaying the progression of the disease, increasing the quality of life, and/or prolonging survival). The disease herein is cancer.

"Prevention" or "prophylaxis" refers to the treatment of a disease or condition that causes the clinical symptoms of the disease or condition not to develop or progress. Pharmaceutical compositions may, in some embodiments, be administered to a subject (including a human) who is at risk or has a family history of the disease or condition in order to prevent the disease or condition. The disease herein is cancer, preferably a cancer expressing the cysteine/glutamate antiporter xCT. In certain aspects, the cancer expressing the cysteine/glutamate antiporter xCT is p53 negative.

An "isomer" or "stereoisomer" of a D-cysteine, or a derivative thereof, refers to a cysteine made up of the same atoms bonded by the same bonds but having different three-dimensional structures, which are not interchangeable. The present invention contemplates various stereoisomers and mixtures thereof and includes "enantiomers," which refers to two stereoisomers whose molecules are non-superimposable mirror images of one another. "Diastereomers" are stereoisomers that have at least two asymmetric atoms, but which are not mirror-images of each other. L-cysteine is excluded from the present invention even though it is considered an isomer of D-cysteine.

Compositions and pharmaceutical compositions provided herein that include a D-cysteine described herein, a derivative or salt, isomer, or a mixture thereof may include racemic mixtures, or mixtures containing an enantiomeric excess of one enantiomer or single diastereomers or diastereomeric mixtures. All such isomeric forms of this D-cysteine, salt, isomer, derivative or a mixture thereof are expressly included herein the same as if each and every isomeric form were specifically and individually listed.

The D-cysteine or derivative thereof, as well as the isomer of a D-cysteine of the present disclosure, can be in the form of a salt, preferably in the form of a pharmaceutically acceptable salt. The term "pharmaceutically acceptable salts" refers to salts prepared from pharmaceutically acceptable non-toxic bases or acids, including inorganic bases or acids and organic bases or acids. In case the D-cysteine, isomer, or derivative thereof of the present disclosure contain one or more acidic or basic groups, the disclosure also comprises their corresponding pharmaceutically or toxicologically acceptable salts, in particular their pharmaceutically utilizable salts. Thus, the D-cysteine or derivative thereof, isomer of the present disclosure which contain acidic groups can be present on these groups and can be used according to the disclosure, for example, as alkali metal salts, alkaline earth metal salts or ammonium salts. More precise examples of such salts include sodium salts, potassium salts, calcium salts, magnesium salts or salts with ammonia or organic amines such as, for example, ethylamine, ethanolamine, triethanolamine or amino acids. The D-cysteine, isomer, or derivative thereof of the present disclosure which contain one or more basic groups, i.e. groups which can be protonated, can be present and can be used according to the disclosure in the form of their addition salts with inorganic or organic acids. Examples of suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the compounds of the present disclosure simultaneously contain acidic and basic groups in the molecule, the disclosure also includes, in addition to the salt forms mentioned, inner salts or betaines (zwitterions). The respective salts can be obtained by customary methods which are known to the person skilled in the art like, for example, by contacting these with an organic or inorganic acid or base in a solvent or dispersant, or by anion exchange or cation exchange with other salts. The present disclosure also includes all salts of the D-cysteine, isomer, or derivative thereof of the present disclosure which, owing to low physiological compatibility, are not directly suitable for use in pharmaceuticals but which can be used, for example, as intermediates for chemical reactions or for the preparation of pharmaceutically acceptable salts.

A "D-cysteine derivative" refers to, e.g. a non-proteinogenic amino acid derivative resulting from the formal reaction of D-cysteine at the amino group, carboxy group, or from the replacement of any hydrogen of D-cysteine by a heteroatom or to an oligomer (e.g. dimer, trimer, etc...) of one or more thereof.

Preferably, the D-cysteine derivative is selected from the group comprising D-cystine, homocysteine, meso-lanthionine, S-(2-aminovinyl)-3-methyl-D-cysteine, S-(2-aminovinyl)-D-cysteine and n-acetyl-D-cysteine or a combination of one of more thereof. Most preferably, the the D-cysteine derivative is D-cystine. Salts and isomers of one or more derivatives of D-cysteine are also encompassed in the present invention.

The composition or pharmaceutical composition of the invention further comprises a pharmaceutically acceptable carrier and/or diluent.

"Pharmaceutically acceptable carrier and/or diluent" means a carrier and/or diluent that is useful in preparing a pharmaceutical composition that is generally safe, non-toxic, and desirable, and includes carriers or diluents that are acceptable for human pharmaceutical use.

Such pharmaceutically acceptable carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions.

Pharmaceutically acceptable excipients include starch, glucose, lactose, sucrose, sodium stearate, glycerol monostearate, talc, sodium chloride, dried skim milk, glycerol, propylene glycol, water, ethanol and the like.

Additionally, auxiliary substances, such as wetting or emulsifying agents, pH buffering substances, gels or gelling materials, flavorings, colorants, microspheres, polymers, suspension agents, etc. may also be present herein. In addition, one or more other conventional pharmaceutical ingredients, such as preservatives, humectants, suspending agents, surfactants, antioxidants, anticaking agents, fillers, chelating agents, coating agents, chemical stabilizers, etc. may also be present, especially if the dosage form is a reconstitutable form. Suitable exemplary ingredients include macrocrystalline cellulose, carboxymethyl cellulose sodium, polysorbate 80, phenylethyl alcohol, chlorobutanol, potassium sorbate, sorbic acid, sulfur dioxide, propyl gallate, the parabens, ethyl vanillin, glycerin, phenol, parachlorophenol, gelatin, albumin and a combination thereof. A thorough discussion of pharmaceutically acceptable excipients is available in REMINGTON'S PHARMACEUTICAL SCIENCES (Mack Pub. Co., N.J. 1991) which is incorporated by reference herein.

As used herein, the "cancer" is usually selected from the group comprising solid cancers, lymphomas and leukemias. Preferably, the cancer is a solid cancer, lymphoma or leukemia expressing the cysteine/glutamate antiporter xCT. Metastatic cancer is also within the scope of this invention.

The cysteine/glutamate antiporter xCT refers to an amino acid antiporter that typically mediates the exchange of extracellular cystine and intracellular glutamate across the cellular plasma membrane. This antiporter is highly expressed in several types of human cancer (Lewerenz J., Hewett S. J., Huang Y., Lambros M., Gout P. W., Kalivas P. W., Massie A., Smolders I., Methner A., Pergande M., Smith S. B., Ganapathy V., and Maher P. (2013) The cystine/glutamate antiporter system xc- in health and disease: from molecular mechanisms to novel therapeutic opportunities. Antioxid. Redox Signal. 18, 522-555; Bhutia Y. D., Babu E., Ramachandran S., and Ganapathy V. (2015) Amino acid transporters in cancer and their relevance to "glutamine addiction": novel targets for the design of a new class of anticancer drugs. Cancer Res. 75, 1782-1788).

Preferably, the cancer expressing the cysteine/glutamate antiporter xCT is selected from the non-limiting group comprising carcinoma, blastoma, sarcoma, mesothelioma, melanoma, lymphoma, leukemia and lymphoid malignancies or a combination of one of more thereof.

Examples of cancers expressing the cysteine/glutamate antiporter xCT comprise bladder cancer, liver cancer, colon cancer, rectal cancer, endometrial cancer, leukemia, lymphoma, pancreatic cancer, small cell lung cancer, non-small cell lung cancer, breast cancer, urethral cancer, head and neck cancer, gastrointestinal cancer, stomach cancer, malignant pleural mesothelioma, malignant peritoneal mesothelioma, oesophageal cancer, ovarian cancer, renal cancer, melanoma, prostate cancer and thyroid cancer, or a combination of one of more thereof.

The compositions and pharmaceutical compositions of the invention may be suitable for oral, rectal, topical, parenteral (including subcutaneous, intramuscular, and intravenous), ocular (ophthalmic), pulmonary (nasal or buccal inhalation) or nasal administration, although the most suitable route in any given case will depend on the nature and severity of the cancer being treated. They may be conveniently presented in unit dosage form and prepared by any of the methods well-known in the art of pharmacy.

Because of their ease of administration, tablets and capsules represent the most advantageous oral dosage unit form in which case solid pharmaceutical carriers are employed. If desired, tablets may be coated by standard aqueous or non-aqueous techniques. Such compositions and preparations should contain at least 0.1 percent of D-cysteine or a derivative thereof, an isomer or a salt of said D-cysteine or derivative (hereafter the active compound). The percentage of active compound in these compositions may, of course, be varied and may conveniently be between about 2 percent to about 60 percent of the weight of the unit. The amount of active compound in such therapeutically useful compositions is such that an effective dosage will be obtained. The compositions of the invention can also be administered, as stated elsewhere, intranasally as, for example, liquid drops or spray.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

Since salt forms of ionic compounds can substantially affect bioavailability, the compounds of the present disclosure may also be used as salts with various counterions to yield an orally available formulation. Pharmaceutically acceptable counterions may be mono- or bivalent ions such as ammonium, the alkaline metals sodium or potassium or the alkaline earth metals magnesium or calcium, certain pharmaceutically acceptable amines such as tris(hydroxymethyl)aminomethane, ethylendiamine, diethylamine, piperazine or others, or certain cationic amino acids such as lysine or arginine.

The compounds of the present disclosure may also be administered parenterally. Solutions or suspensions of these active compounds can be prepared in water suitably mixed with a surfactant such as hydroxy-propylcellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oils.

Any suitable route of administration may be employed for providing a mammal, especially a human, with an effective dose of a compound of the present disclosure. For example, oral, rectal, topical, parenteral, ocular, pulmonary, nasal, and the like may be employed. Dosage forms include tablets, troches, dispersions, suspensions, solutions, capsules, creams, ointments, aerosols, and the like. In some embodiments, compounds of the present disclosure are administered orally.

The pharmaceutical compositions of the invention may be administered alone or substantially simultaneously or concurrently with one or more additional therapeutic agent or therapy.

Such additional therapeutic agent or therapy is preferably selected from the group comprising a D-amino acid oxidase (DAAO) inhibitor, an anticancer agent or therapy, an iron chelator, or a combination of one of more thereof.

D-Amino acid oxidase (DAAO) is a flavin adenine dinucleotide (FAD)-containing flavoenzyme that catalyzes with absolute stereoselectivity the oxidative deamination of all natural D-amino acids, the only exception being the acidic ones. This flavoenzyme plays different roles during evolution and in different tissues in humans (Pollegioni L, Sacchi S, Murtas G. Human D-Amino Acid Oxidase: Structure, Function, and Regulation. Front Mol Biosci. 2018;5:107. Published 2018 Nov 28.).
Examples of DAAO) inhibitors include those selected from the group comprising a substrate-competitive DAAO inhibitor, a FAD-competitive DAAO inhibitor and a substrate- and FAD-competitive inhibitor, or a combination of one of more thereof.

Preferably, the DAAO inhibitor is selected from the group comprising 3-substituted 5-hydroxy-1,2,4-triazin-6(1H)-one, benzoate (e.g. sodium benzoate), o-aminobenzoate, anthranilate, substituted quinilinones, 4H-furo[3,2-b]pyrrole-5-carboxylic acid, 6-Chloro-1,2-benzisoxazol-3(2H)-one (CBIO), Adenosine diphosphate, Chlorpromazine, and 2-(2,5-dimethylphenyl)-6-fluorobenzo[d]isothiazol-3(2H)-on, 6-hydroxy-3-phenethyl-1,2,4-triazin-5(2H)-one, 3-((6-fluoronaphthalen-2-yl)methylthio)-6-hydroxy-1,2,4-triazin-5(2H)-one, JHU 1057, and JHU 1377, TAK-831, or a derivative or combination of one or more thereof.

Additional DAAO inhibitor examples are available, e.g. in Hin N, Duvall B, Berry JF, et al. D-Amino acid oxidase inhibitors based on the 5-hydroxy-1,2,4-triazin-6(1H)-one scaffold. Bioorg Med Chem Lett. 2016;26(8):2088-2091, as well as in WO2013004996 (Takeda), WO2013004995 (Takeda), WO2014096757 (Takeda), US2019112289 (Syneurx International), WO2019228408 (Syneurx International)which are all incorporated by reference herein.

Examples of an anticancer agent or therapy include those selected from the group comprising surgery, radiotherapy, chemotherapy, immunotherapy, hormone therapy, an immunostimulatory therapy, a cellular therapy, and a gene therapy, or a combination of one of more thereof.

A chemotherapy of the present invention refers to agents that damage DNA and / or prevent cells from multiplying, such as genotoxins.

Genotoxins can be selected from the group comprising alkylating agents, antimetabolites, DNA cutters, DNA binders, topoisomerase poisons and spindle poisons, or a combination of one of more thereof.

Non-limiting examples of alkylating agents are selected from the group comprising lomustine, carmustine, streptozocin, mechlorethamine, melphalan, uracil nitrogen mustard, chlorambucil, cyclosphamide, iphosphamide, cisplatin, carboplatin, mitomycin, thiotepa, dacarbazin, procarbazine, hexamefhyl melamine, triethylene melamine, busulfan, pipobroman, mitotane and other platine derivatives, or a combination of one of more thereof.

An example of DNA cutters is bleomycin.
Topoisomerases poisons can be selected from the group comprising topotecan, irinotecan, camptothecin sodium salt, daorubicin, doxorubicin, idarubicin, mitoxantrone teniposide, adriamycin and etoposide, or a combination of one of more thereof.

Non-limiting examples of DNA binders are selected from the group comprising dactinomycin and mithramycin whereas spindle poisons can be selected among the group comprising vinblastin, vincristin, navelbin, paclitaxel and docetaxel, or a combination of one of more thereof.

A chemotherapy of the present invention can concern as well antimetabolites selected among the group comprising methotrexate, trimetrexate, pentostatin, cytarabin, ara-CMP, fludarabine phosphate, hydroxyurea, fluorouracyl, fioxuridine, chlorodeoxyadenosine, gemcitabine, thioguanine and 6-mercaptopurine, or a combination of one of more thereof.

A radiotherapy of the present invention refers to the use of high-energy radiation to shrink tumors and kill cancer cells. Examples of radiation therapy include, without limitation, external radiation therapy and internal radiation therapy (also called brachytherapy).

Examples of an immunotherapy are selected from the group comprising immune checkpoint inhibitors (e.g. against check-point proteins PD1 and/or PD-L1, CTLA-4), T-cell transfer therapy (e.g. tumor-infiltrating lymphocytes therapy and CAR T-cell therapy), antibodies (e.g. monoclonal antibodies, targeted antibody drug conjugates, ...), treatment vaccines and immune system modulators.

Gene therapy and cellular therapy include the insertion of a normal gene into cancer cells to replace a mutated or altered gene; genetic modification to silence a mutated gene; genetic approaches to directly kill the cancer cells; including the infusion of immune cells designed to replace most of the patient's own immune system to enhance the immune response to cancer cells, or activate the patient's own immune system (T cells or Natural Killer cells) to kill cancer cells, or find and kill the cancer cells; genetic approaches to modify cellular activity to further alter endogenous immune responsiveness against cancer. The genome editing system is usually selected from the group comprising a CRISPR/Cas9 system, a zinc finger nuclease system, a TALEN system, a homing endonucleases system, and a meganuclease system.

In an particular aspect, gene therapy includes the insertion of *SLC7A11* and/or *SLC3A2* genes, which encode the two components of the xCT cystine/glutamate antiporter, in cancer cells that do not express, or under express, the xCT cystine/glutamate antiporter. Preferably, the *SLC7A11* and/or *SLC3A2* gene will be under the control of one or more regulatory elements (e.g. inducible promoter or enhancer) to enable its/their overexpression.

In another aspect, gene therapy includes the insertion of one or more regulatory elements (e.g. inducible promoter or enhancer) close to the *SLC7A11* and/or *SLC3A2* genes in cancer cells that do not express, or under express, the xCT cystine/glutamate antiporter so as to enable or induce its overexpression.

In a further aspect, gene therapy includes the insertion of one or more regulatory elements (e.g. inducible promoter or enhancer) close to one or more candidate genes that are most implicated in D-cysteine toxicity, in cancer cells, so as to enable or induce its/their overexpression. Preferably, the one or more candidate genes that are most implicated in D-cysteine toxicity are selected from the group of genes listed in Fig. 6.

In still another aspect, gene therapy includes the administration of a gene delivery vector, preferably in the form of a plasmid or a vector, which comprises one or more nucleic acid(s) encoding the *SLC7A11* and/or *SLC3A2* genes in cancer cells that do not express, or under express, the xCT cystine/glutamate antiporter so as to enable or induce its/their overexpression. Preferably, said vector is a viral vector suited for ex-vivo and in-vivo gene delivery. More preferably, the viral vector is selected from the group comprising an adeno-associated virus (AAV) and a lentivirus, e.g. Lentivirus of 1st, 2nd, and 3rd generation, not excluding other viral vectors such as adenoviral vector, herpes virus vectors, etc.... Other means of delivery or vehicles are known (such as yeast systems, microvesicles, microemulsions, gene guns/means of attaching vectors to gold nanoparticles) and are provided herein. In some aspects, one or more of the viral or plasmid vectors may be delivered via liposomes, micro- or nanoparticles, exosomes, microvesicles, or a gene-gun.

An iron chelator of the invention can be a synthetic or a natural iron chelator. Examples of iron chelators are selected from the non-limiting group comprising deferoxamine and its derivatives, baicalin, ligustrazine, quercetin, epigallocatechin gallate, theaflavin, phytic acid and genistein, salts thereof or a combination of one of more thereof. Examples of deferoxamine derivatives comprise those described in Yu, Yu & Wong, Jacky & Lovejoy, David & Kalinowski, Danuta & Richardson, Des. (2007). Chelators at the Cancer Coalface: Desferrioxamine to Triapine and Beyond. Clinical cancer research: an official journal of the American Association for Cancer Research, which is incorporated by reference herein.

It will be appreciated that the combination of a pharmaceutical composition of the invention and the one or more additional therapeutic agent or therapy may be administered in any order or concurrently. In selected aspects, the pharmaceutical composition and the one or more additional therapeutic agent or therapy will be administered to patients that have previously undergone treatment with other anti-cancer agents. In certain other aspects, the pharmaceutical composition and the one or more additional therapeutic agent or therapy will be administered substantially simultaneously or concurrently. For example, a subject may be given a pharmaceutical composition of the invention while undergoing a course of treatment with the one or more additional therapeutic agent or therapy. In addition, it is contemplated that the subject has already or may be concurrently receiving other forms of cancer therapy, e.g., chemotherapy. In certain aspects, the pharmaceutical composition of the invention will be administered within 1 year of the treatment with the one or more additional therapeutic agent or therapy. In certain alternative aspects, the pharmaceutical composition of the invention will be administered within 10, 8, 6, 4, or 2 months of any treatment with the one or more additional therapeutic agent or therapy (e.g. anti-cancer therapy). In certain other aspects, the pharmaceutical composition of the invention will be administered within 4, 3, 2, or 1 week of any treatment with the one or more additional therapeutic agent or therapy (e.g. additional anti-cancer agent or therapy). In some aspects, the pharmaceutical composition of the invention will be administered within 5, 4, 3, 2, or 1 days of any treatment with the one or more additional therapeutic agent or therapy. It will further be appreciated that the pharmaceutical composition of the invention and the one or more additional therapeutic agent or therapy may be administered to the subject within a matter of hours or minutes (i.e., substantially simultaneously).

In another aspect, the composition or pharmaceutical composition comprising a D-cysteine or a derivative thereof, an isomer or a salt of said D-cysteine or derivative is for use in the treatment and/or prevention of a ferrotoxic disease or an iron related disease or disorder. The rationale for these therapeutic indications is that D-cysteine acts as an iron chelator in the cell (Figure 3).

These include hereditary or secondary hemochromatosis, African iron overload, sickle cell disease, thalassemia, X-linked sideroblastic anemia, and very rare protein transport disorders such as aceruloplasminemia or a transferrinemia.

The pharmaceutical compositions of the invention for use in the treatment and/or prevention of a ferrotoxic disease or an iron related disease or disorder may be administered alone or substantially simultaneously or concurrently with one or more additional therapeutic agent or therapy. Such additional therapeutic agent or therapy is preferably selected from the group comprising a D-amino acid oxidase (DAAO) inhibitor, an iron chelator, a cellular therapy, and a gene therapy, or a combination of one of more thereof.

In this aspect, gene therapy includes the administration of a gene delivery vector, preferably in the form of a plasmid or a vector, which comprises one or more nucleic acid(s) encoding the *SLC7A11* and/or *SLC3A2* genes in liver cells that do not express, or under express, the xCT cystine/glutamate antiporter so as to enable or induce its/their overexpression. Preferably, the *SLC7A11* and/or *SLC3A2* gene, or the gene delivery vector itself (e.g. the adenovirus vector), will be under the control of one or more liver-specific regulatory elements (e.g. liver-specific promoter or enhancer) to enable its/their overexpression specifically in liver cells, which are among the most affected cells in ferrotoxic pathologies.

The present invention provides, in a further aspect, the use of a composition or pharmaceutical composition in the preparation of a medicament for the treatment and/or prevention of a ferrotoxic disease, an iron related disease or disorder, or a cancer.

The present invention further provides a method of treatment and/or prevention of a cancer (including cancer metastasis) expressing the cysteine/glutamate antiporter xCT in a subject in need thereof, comprising administering to said subject a pharmaceutical composition of the invention, alone or in combination with one or more additional therapeutic agent or therapy. As discussed above, the cancer is, in certain aspects, p53 negative.

Such additional therapeutic agent or therapy is preferably selected from the group comprising a D-amino acid oxidase (DAAO) inhibitor, an anticancer agent or therapy, an iron chelator, or a combination of one of more thereof, as described herein.

Further provided is a pharmaceutical composition comprising a D-cysteine or a derivative thereof, an isomer or a salt of said D-cysteine or derivative, for use in a method for the treatment and/or prevention of a cancer in a patient, wherein the method comprises: (i) determining whether the cancer from the patient expresses the cysteine/glutamate antiporter xCT; and (ii) if the cancer from the patient expresses said cysteine/glutamate antiporter xCT, administering to the patient an effective amount of the pharmaceutical composition.

Also provided is a method of treatment and/or prevention of a ferrotoxic disease, or an iron related disease or disorder, in a subject in need thereof, comprising administering to said subject a pharmaceutical composition of the invention, alone or in combination with one or more additional therapeutic agent or therapy.

Further provided is a method for decreasing the proliferation of a cancer cell, preferably a cancer (including cancer metastasis) cell expressing the cysteine/glutamate antiporter xCT, comprising contacting said cancer cell with a composition or pharmaceutical composition of the invention, alone or in combination with one or more additional therapeutic agent or therapy. In certain aspects, the cancer cell is a p53 negative cancer cell.

In certain aspects, the method comprises decreasing the proliferation of a cancer cell (or a metastatic cancer cell), by at least 5%, by at least 10%, by at least 15%, by at least 20%, by at least 25%, by at least 30%, by at least 35%, by at least 40%, by at least 45%, by at least 50%, or more as compared to a control assay in the absence of a composition, or pharmaceutical composition, of the invention.

Also provided is a method for enhancing the ferritinophagy in a cancer cell, preferably a cancer (including cancer metastasis) cell expressing the cysteine/glutamate antiporter xCT, comprising contacting said cancer cell with a composition or pharmaceutical composition of the invention, alone or in combination with one or more additional therapeutic agent or therapy. In certain aspects, the cancer cell is a p53 negative cancer cell.

In certain aspects, the method comprises enhancing the ferritinophagy in a cancer cell (or a metastatic cancer cell), by at least 5%, by at least 10%, by at least 15%, by at least 20%, by at least 25%, by at least 30%, by at least 35%, by at least 40%, by at least 45%, by at least 50%, or more as compared to a control assay in the absence of a composition, or pharmaceutical composition, of the invention.

Further provided is a method of treatment and/or prevention of a cancer in a patient, wherein the method comprises: (i) determining whether the cancer from the patient expresses the cysteine/glutamate antiporter xCT; and (ii) if the cancer from the patient expresses said cysteine/glutamate antiporter xCT, administering to the patient an effective amount of the pharmaceutical composition of the invention.

While certain features of this invention have been illustrated and described herein, many modifications, substitutions, changes, and equivalents will now occur to those of ordinary skill in the art. It is, therefore, to be understood that the appended claims are intended to cover all such modifications and changes as fall within the true spirit of this invention.

### EXAMPLES

### D-cysteine affects tumor cell viability

Research funded by our current research grant from Oncosuisse into the role of pyruvate metabolism on oxygen consumption (OCR), showed that OCR of cells cultured in a poor medium containing only glucose and supplemented with amino acids (except glutamine) displayed a lower oxygen consumption rate than cells cultured in medium containing glucose alone. We therefore explored this apparent inhibitory effect of amino acids on mitochondrial respiration by testing each amino acid separately and found that L-cysteine was responsible for the observed effect (data not shown). As a control, we used the D-cysteine enantiomer and to our surprise we found a similar effect suggesting that inhibition of OCR is most likely mediated by the thiol group rather than the 3-D structure of the molecule.

We therefore decided to test whether the D-cysteine-mediated inhibition of OCR could affect cell growth in a regular normal medium complemented with fetal calf serum. Most culture media used for cell culture contain 300 µM L-cystine. We observed that addition of either 500 µM D-cystine or 500 µM D-cysteine to the culture medium inhibited the proliferation of A549 cells, whereas addition of 500 µM L cysteine had no effect (Figure 1). The antiproliferative effect of D-cysteine was found with many cancer cell lines including LuCa62 and H1299 lung adenocarcinoma cells, BZR tumorigenic bronchial epithelial cells, the MDA-MB-231 highly aggressive and poorly differentiated triple-negative breast cancer cells, A375 melanoma cells, and different cell lines derived from malignant pleural mesothelioma (MPM), whether the cells were cultured in colony formation assays or in spheroids (Figure 4 and data not shown). Importantly, some cancer cell lines, including HeLa cells (Figure 4A), the MPM cell line H2052/484 (figure 4B), and osteosarcoma cell line 143B (not shown), were insensitive both in 2D cultures or in colony formation assays. The decrease in numbers of cells seen after 72 h exposure to D-cysteine was due to decreased cell proliferation. After 72 hours, following the first passage of cells ("p1"), extensive cell death was observed (Figure 5A). No increase in caspase 3 could be detected by Western blotting and addition of the pan-caspase inhibitor zVAD-fmk did not protect the cells suggesting that cell death was not through apoptosis (data not shown). Importantly, in contrast to the results with cancer cells, normal human bronchial epithelial BEAS-2B cells were resistant to D-cysteine exposure even at very high concentration (Figure 5B).

As described later, the cell-type specificity of D-cysteine is related to their ability to import D-cysteine.

### Molecular mechanisms underlying D-cysteine toxicity

Two experimental approaches were pursued in an attempt to understand how D-cysteine exerts its toxic effect, i) A CRISPR/Cas9 genome wide screen to identify genes which when inactivated could suppress D-cysteine toxicity, and ii) a proteomic approach to investigate the effects of D-cysteine on gene expression.

### Genes mediating the toxic effect of D-cysteine

For these experiments a pooled lentiviral CRISPR library (Addgene) targeting 19114 human genes was used to infect A549 cells, stably expressing the Cas9 nuclease. Cells were then cultured in the presence of 0.5 mM D-cysteine, a concentration sufficient to induce A549 cell death. After 2-3 weeks, the surviving clones were recovered and the sgRNAs were amplified by PCR and sequenced using next-generation sequencing (NGS) to identify the targeted genes (Figure 6).

### Cystine-glutamate antiporter (xCT)

The two most significant hits to emerge from the screen (Figure 6B) were *SLC7A11* and *SLC3A2,* which encode the two components of the xCT cystine/glutamate antiporter. This suggests that the xCT antiporter provides the mechanism by which D-cysteine is imported into cells, and this was confirmed in a colony assay in which each of the two genes were individually ablated (Figure 7). At this point there are two options, either D-cysteine is oxidized in the extracellular medium and then internalized through the xCT as D-cystine, or in addition to D-cystine, the carrier is also able to transport D-cysteine. Experiments are in progress to distinguish between these two possibilities. Importantly in the current context, it is interesting to note that while this carrier is poorly expressed by normal cells, it is known to be highly expressed in many cancer cells³⁵.

In addition to the xCT carrier, the most potent hits included genes involved in iron transport and ferritinophagy .

We first tested whether D-cysteine could trigger ferroptosis. Ferroptosis is a type of death due to iron-mediated ROS production and lipid peroxidation. In most cases, it is triggered by decreased glutathione levels. We measured glutathione levels and found that in contrast to other activators of ferroptosis which block the xCT transporter, D-cysteine did not induce a drop in glutathione (Figure 8A). However, like other mediators of ferroptosis, we observed that D-cysteine induced ROS production (Figure 8B) and lipid peroxidation (Figure 8C). This suggests that D-cysteine activates the last step of ferroptosis.

Moreover, D-cysteine could also trigger ferritinophagy, which is a process triggered in cells lacking iron. The most likely explanation is that D-cysteine, which accumulates in cells since it is not consumed during protein synthesis or other pathways, can react with iron through its thiol group, thus acting as an iron chelator. This is consistent with the decrease in cellular iron assessed using ICP-MS in cells treated with D-cysteine (Figure 3). As a consequence intracellular iron is depleted, and cells trigger ferritinophagy releasing ferric iron, thus leading to ROS production, lipid peroxidation and cell death.

### Proteomic analysis of cells exposed to D-cysteine

To understand how D-cysteine modifies protein expression, A549 cells were grown in the presence of D-cysteine for 3 days and total cellular proteins were compared to control cells grown in parallel in the presence of L-cysteine. For these experiments liquid chromatography-tandem mass spectrometry (LC-MS/MS) coupled with tandem mass tagging (TMT) was employed, a procedure which enables simultaneous protein identification and relative quantification of two different samples in the same proteomics experiment.

Interestingly, in the presence of D-cysteine (Table 1) we observed altered expression of many components of mitochondrial complex I, the NADH-ubiquinone oxidoreductase complex of the electron transport chain (ETC). Most of the subunits which make up this large enzyme complex (NDUFA5, NDUFA7, NDUFA9, NDUFA10, NDUFA12, NDUFS1, NDUFS4, NDUFS7, NDUFS8, NDUFV1 NDUFV2, MT-ND4) were found to be down-regulated in D-cysteine-treated cells.

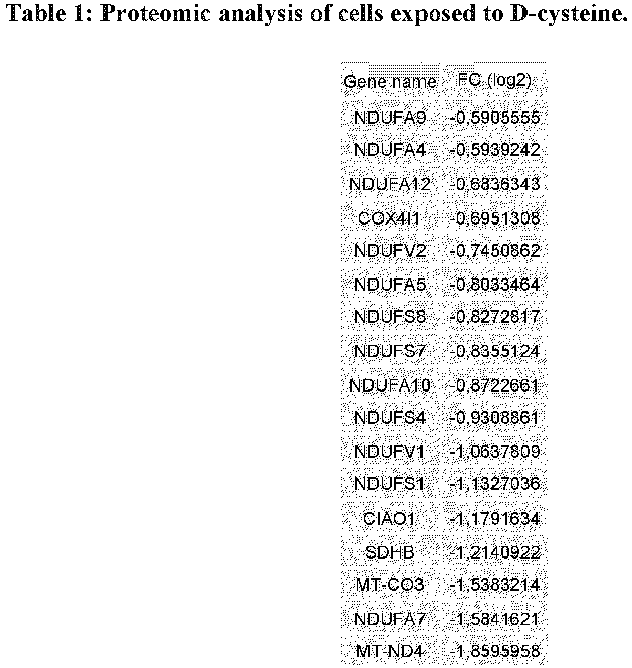

Proteome analysis of A549 cells cultured in the absence or presence of 500 µM D-cysteine for 72 h. The analysis revealed that many proteins involved in mitochondrial respiration are down-regulated following exposure to D-cysteine. FC=Fold change. FDR (False discovery rate) < 0.01

As described above our data have implicated ferritinophagy and lipid peroxidation in D-cysteine toxicity and it is of interest that lipid peroxidation has been shown to disrupt the formation of Complex I (CI)⁴².

Analysis of mitochondrial respiration in A549 lung tumor cells showed that D-cysteine strongly impaired basal and maximal oxygen consumption (Figure 9), totally consistent with the observed changes in the protein composition of CI.

These results to date are consistent with the hypothesis that D-cysteine is toxic for cells as a result of changes in iron metabolism, and only those cells that can import sufficient amounts of this D-cysteine through the xCT transporter would be affected. From our results we conclude that D-cysteine is acting at least in part as an iron chelator. Importantly, unlike small molecule iron chelators, D-cysteine shows selective toxicity for many cancer cells since the cystine/glutamate antiporter, the route through which D-cysteine enters the cell, is frequently upregulated in cancer cells. Small molecule iron chelators on the other hand, would be unable to discriminate between normal and cancer cells.

### Pharmacokinetics of D-cysteine

As described below, we have initiated experiments in which we administer D-cysteine (500 mg/kg) by gavage, and using the technique described in ³⁷, we have measured the levels of D-cysteine in serum. We found that the initial level of D-cysteine in the blood is around 200 µM, and it dropped to 5 µM within the first 2 h (data not shown). We reasoned that the enzyme D amino oxidase (DAAO), which is known to oxidize D-amino acids and which in mice is highly expressed in the kidney, could explain the rapid clearance of D-cysteine from the blood. We therefore co-administered D-cysteine with an inhibitor of DAAO (JHU 1377). Using this protocol, we achieved higher levels of D-cysteine in the blood (Table 2) and indeed levels higher than that needed to repress tumor cell growth *in vitro.* Thus far we have only assessed the pharmacokinetics of D-cysteine during the first 6 h following administration. In future experiments we plan to repeat the analysis over longer time periods.

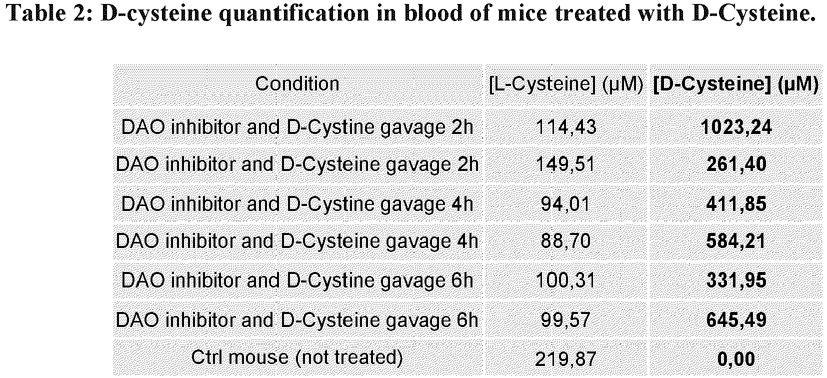

DAAO inhibitor JHU 1377 (30 mg/kg) was injected into mice, followed 2 h later by D-cysteine gavage (500 mg/kg). Blood was collected after 2, 4, and 6 h and levels of L- and D-cysteine were quantified in the serum.

### Toxicity

We plan to administer D-cysteine, once or twice a day depending on the results of the pharmacokinetics studies, and to monitor any adverse effects. Similar experiments have been previously performed in the rat⁴⁴. The purpose of the experiments in the rat was to compare the relative toxicity of L-cysteine and D-cysteine and the authors found that administration of D-cysteine at 500 mg/kg daily by gavage for one month had no adverse effect on the animals. These results are encouraging, however we need to confirm them in the mouse especially since we also plan to include the DAAO inhibitor to achieve longer serum half-life of D-cysteine.

### Anticancer efficacy of D-cysteine in mouse models of lung cancer

We plan to study two types of cancer cells against which D-cysteine has shown efficacy *in vitro:* A549 and LuCa62 cells for lung cancer, and JL1 and ZL34, which are highly aggressive mesothelioma cells. Each cell line will be used to generate xenografts or orthotopic cancers. First the effect of D-cysteine will be assessed in classical sub-cutaneous xenograft models in immunocompromised mice that are easy to monitor and provide strong data on the comportment and response of the target tumor. However, the sub-cutaneous environment and the phenotype of the sub-cutaneous cells surrounding the tumors (fibroblasts, leucocytes, macrophages) will not be comparable to those found in the original tumor tissue derived from the thoracic cavity and lung⁴⁵. The stromal cells secrete growth factors and cytokines which contribute to the tumor microenvironment and can modulate tumor growth⁴⁶. Therefore, in the second model, the effect of D-cysteine will be assessed in orthotopic xenograft models of human mesothelioma and lung carcinoma cells in immunocompromised mice. In the case of lung adenocarcinoma, tumor cells will be transplanted into the lung parenchyma⁴⁷. The development of the tumors in control mice and mice treated with D-cysteine will be monitored by (¹⁸F)FDG-PET/CT imaging once per week, starting on day 7 after implantation of cancer cells. When committee-approved endpoints are reached, the mice will be euthanized and tumor growth will be assessed. For each euthanized mouse, blood and pleural fluid will be collected for analysis of D-cysteine and cytokine levels; spleen, lung, thoracic tumors, and brain will be explanted. Part of each sample will be fixed in 10% neutral buffered formalin for histology and immunohistochemistry. The rest will be stored in -80°C for subsequent molecular biology analysis.

### Significance of the planned work

Amino acids provide substrates for new protein synthesis, and in rapidly proliferating cells such as tumor cells they are in especially high demand. In addition, cysteine is required for multiple cellular processes that rely on the chemistry of its thiol group. Despite being a non-essential amino acid, cysteine is imported into cells by the xCT transporter in the plasma membrane, and indeed the xCT pathway is highly upregulated in many forms of cancer including lung cancer. Moreover, high levels of xCT expression correlate with poor prognosis in lung cancer. The initial experiments described here show that D-cysteine is a substrate for xCT-mediated transport and leads to selective toxicity towards cancer cells in culture. The key question now is to evaluate the activity of D-cysteine in *in vivo* models of lung cancer.

### REFERENCES

1 Mossman, B. T. & Gee, J. B. Asbestos-related diseases. N Engl J Med 320, 1721-1730, doi:10.1056/NEJM198906293202604 (1989).
2 Pass, H. I. et al. Asbestos exposure, pleural mesothelioma, and serum osteopontin levels. N Engl J Med 353, 1564-1573, doi:10.1056/NEJMoa051185 (2005).
3 Glynn, M. E., Keeton, K. A., Gaffney, S. H. & Sahmel, J. Ambient Asbestos Fiber Concentrations and Long-Term Trends in Pleural Mesothelioma Incidence between Urban and Rural Areas in the United States (1973-2012). Risk Anal 38, 454-471, doi:10.1111/risa.12887 (2018).
4 Zhao, J. et al. Epidemiology and trend analysis on malignant mesothelioma in China. Chin J Cancer Res 29, 361-368, doi:10.21147/j.issn.1000-9604.2017.04.09 (2017).
5 Robinson, B. W., Musk, A. W. & Lake, R. A. Malignant mesothelioma. Lancet 366, 397-408, doi:10.1016/S0140-6736(05)67025-0 (2005).
6 Belalcazar, A. D., Ball, J. G., Frost, L. M., Valentovic, M. A. & Wilkinson, J. t. Transsulfuration Is a Significant Source of Sulfur for Glutathione Production in Human Mammary Epithelial Cells. ISRNBiochem 2013, 637897, doi:10.1155/2013/637897 (2014).
7 Zhang, W. et al. Stromal control of cystine metabolism promotes cancer cell survival in chronic lymphocytic leukaemia. Nat Cell Biol 14, 276-286, doi:10.1038/ncb2432 (2012).
8 Fotiadis, D., Kanai, Y. & Palacin, M. The SLC3 and SLC7 families of amino acid transporters. Mol Aspects Med 34, 139-158, doi:10.1016/j.mam.2012.10.007 (2013).
9 de la Ballina, L. R. et al. Amino Acid Transport Associated to Cluster of Differentiation 98 Heavy Chain (CD98hc) Is at the Cross-road of Oxidative Stress and Amino Acid Availability. J Biol Chem 291, 9700-9711, doi:10.1074/jbc.M115.704254 (2016).
10 Jiang, L. et al. Ferroptosis as a p53-mediated activity during tumor suppression. Nature 520, 57-62, doi:10.1038/naturel4344 (2015).
11 Eriksson, S., Prigge, J. R., Talago, E. A., Arner, E. S. & Schmidt, E. E. Dietary methionine can sustain cytosolic redox homeostasis in the mouse liver. Nat Commun 6, 6479, doi:10.1038/ncomms7479 (2015).
12 Banjac, A. et al. The cystine/cysteine cycle: a redox cycle regulating susceptibility versus resistance to cell death. Oncogene 27, 1618-1628, doi:10.1038/sj.onc.1210796 (2008).
13 Gorrini, C., Harris, I. S. & Mak, T. W. Modulation of oxidative stress as an anticancer strategy. Nat Rev Drug Discov 12, 931-947, doi:10.1038/nrd4002 (2013).
14 Hakimi, A. A. et al. An Integrated Metabolic Atlas of Clear Cell Renal Cell Carcinoma. Cancer Cell 29, 104-116, doi:10.1016/j.ccell.2015.12.004 (2016).
15 Reznik, E. et al. A Landscape of Metabolic Variation across Tumor Types. Cell Syst 6, 301-313 e303, doi:10.1016/j.cels.2017.12.014 (2018).
16 Pinnix, Z. K. et al. Ferroportin and iron regulation in breast cancer progression and prognosis. Sci Transl Med 2, 43ra56, doi:10.1126/scisignal.3001127 (2010).
17 Dixon, S. J. et al. Ferroptosis: an iron-dependent form of nonapoptotic cell death. Cell 149, 1060-1072, doi:10.1016/j.cell.2012.03.042 (2012).
18 Xie, Y. et al. Ferroptosis: process and function. Cell Death Differ 23, 369-379, doi:10.1038/cdd.2015.158 (2016).
19 Manz, D. H., Blanchette, N. L., Paul, B. T., Torti, F. M. & Torti, S. V. Iron and cancer: recent insights. Ann N Y Acad Sci 1368, 149-161, doi:10.1111/nyas.13008 (2016).
20 Viswanathan, V. S. et al. Dependency of a therapy-resistant state of cancer cells on a lipid peroxidase pathway. Nature 547, 453-457, doi:10.1038/nature23007 (2017).
21 Yang, W. S. et al. Regulation of ferroptotic cancer cell death by GPX4. Cell 156, 317-331, doi:10.1016/j.cell.2013.12.010 (2014).
22 Gao, M. et al. Ferroptosis is an autophagic cell death process. Cell Res 26, 1021-1032, doi:10.1038/cr.2016.95 (2016).
23 Arosio, P., Ingrassia, R. & Cavadini, P. Ferritins: a family of molecules for iron storage, antioxidation and more. Biochim Biophys Acta 1790, 589-599, doi:10.1016/j.bbagen.2008.09.004 (2009).
24 Harrison, P. M. & Arosio, P. The ferritins: molecular properties, iron storage function and cellular regulation. Biochim Biophys Acta 1275, 161-203, doi:10.1016/0005-2728(96)00022-9 (1996).
25 Philpott, C. C. The flux of iron through ferritin in erythrocyte development. Curr Opin Hematol 25, 183-188, doi:10.1097/MOH.0000000000000417 (2018).
26 Kidane, T. Z., Sauble, E. & Linder, M. C. Release of iron from ferritin requires lysosomal activity. Am J Physiol Cell Physiol 291, C445-455, doi:10.1152/ajpcell.00505.2005 (2006).
27 Kishi-Itakura, C., Koyama-Honda, I., Itakura, E. & Mizushima, N. Ultrastructural analysis of autophagosome organization using mammalian autophagy-deficient cells. J Cell Sci 127, 4089-4102, doi:10.1242/jcs.156034 (2014).
28 Mancias, J. D., Wang, X., Gygi, S. P., Harper, J. W. & Kimmelman, A. C. Quantitative proteomics identifies NCOA4 as the cargo receptor mediating ferritinophagy. Nature 509, 105-109, doi:10.1038/nature13148 (2014).
29 Hou, W. et al. Autophagy promotes ferroptosis by degradation of ferritin. Autophagy 12, 1425-1428, doi:10.1080/15548627.2016.1187366 (2016).
30 Mothet, J. P. et al. D-serine is an endogenous ligand for the glycine site of the N-methyl-D-aspartate receptor. Proc Natl Acad Sci U S A 97, 4926-4931, doi:10.1073/pnas.97.9.4926 (2000).
31 Sasabe, J. et al. D-amino acid oxidase controls motoneuron degeneration through D-serine. Proc Natl Acad Sci USA 109, 627-632, doi:10.1073/pnas.1114639109 (2012).
32 Seki, T. et al. d-Cysteine promotes dendritic development in primary cultured cerebellar Purkinje cells via hydrogen sulfide production. Mol Cell Neurosci 93, 36-47, doi:10.1016/j.mcn.2018.10.002 (2018).
36 Chen, D. et al. NRF2 Is a Major Target of ARF in p53-Independent Tumor Suppression. Mol Cell 68, 224-232 e224, doi:10.1016/j.molcel.2017.09.009 (2017).
37 Pucciarini, L. et al. Development and validation of a chiral UHPLC-MS method for the analysis of cysteine enantiomers in biological samples. J Pharm Biomed Anal 177, 112841, doi:10.1016/j.jpba.2019.112841 (2020).
38 Shibata, T. et al. Cancer related mutations in NRF2 impair its recognition by Keap1-Cul3 E3 ligase and promote malignancy. Proc Natl Acad Sci U S A 105, 13568-13573, doi:10.1073/pnas.0806268105 (2008).
39 Singh, A. et al. Dysfunctional KEAP1-NRF2 interaction in non-small-cell lung cancer. PLoS Med 3, e420, doi:10.1371/journal.pmed.0030420 (2006).
40 DeNicola, G. M. et al. Oncogene-induced Nrf2 transcription promotes ROS detoxification and tumorigenesis. Nature 475, 106-109, doi:10.1038/nature10189 (2011).
41 Lien, E. C. et al. Glutathione biosynthesis is a metabolic vulnerability in PI(3)K/Akt-driven breast cancer. Nat Cell Biol 18, 572-578, doi:10.1038/ncb3341 (2016).
42 Lenaz, G. et al. Mitochondrial Complex I: structural and functional aspects. Biochim Biophys Acta 1757, 1406-1420, doi:10.1016/j.bbabio.2006.05.007 (2006).
43 Ferraris, D. et al. Synthesis and biological evaluation of D-amino acid oxidase inhibitors. J Med Chem 51, 3357-3359, doi:10.1021/jm800200u (2008).
44 Shibui, Y., Sakai, R., Manabe, Y. & Masuyama, T. Comparisons of 1-cysteine and d-cysteine toxicity in 4-week repeated-dose toxicity studies of rats receiving daily oral administration. J Toxicol Pathol 30, 217-229, doi:10.1293/tox.2017-0002 (2017).
45 de Jong, M., Essers, J. & van Weerden, W. M. Imaging preclinical tumor models: improving translational power. Nat Rev Cancer 14, 481-493, doi:10.1038/nrc3751 (2014).
46 Devaud, C. et al. Tissues in different anatomical sites can sculpt and vary the tumor microenvironment to affect responses to therapy. Mol Ther 22, 18-27, doi:10.1038/mt.2013.219 (2014).
47 Liu, X. et al. Establishment of an orthotopic lung cancer model in nude mice and its evaluation by spiral CT. J Thorac Dis 4, 141-145, doi:10.3978/j.issn.2072-1439.2012.03.04 (2012).

## Claims

1. A pharmaceutical composition comprising a D-cysteine or a derivative thereof, an isomer or a salt of said D-cysteine or derivative, for use in the treatment and/or prevention of a cancer expressing the cysteine/glutamate antiporter xCT.

2. The pharmaceutical composition for use according to claim 1, further comprising a pharmaceutically acceptable carrier and/or diluent.

3. The pharmaceutical composition for use according to claim 1 or 2, wherein the cancer expressing the cysteine/glutamate antiporter xCT is selected from the group comprising carcinoma, blastoma, sarcoma, mesothelioma , melanoma, lymphoma, leukemia and lymphoid malignancies or a combination of one of more thereof.

4. The pharmaceutical composition for use according to anyone of the preceding claims, wherein the cancer expressing the cysteine/glutamate antiporter xCT is selected from the group comprising bladder cancer, liver cancer, colon cancer, rectal cancer, endometrial cancer, leukemia, lymphoma, pancreatic cancer, small cell lung cancer, non-small cell lung cancer, breast cancer, urethral cancer, head and neck cancer, gastrointestinal cancer, stomach cancer, oesophageal cancer, ovarian cancer, renal cancer, melanoma, malignant pleural mesothelioma, malignant peritoneal mesothelioma, prostate cancer and thyroid cancer, or a combination of one of more thereof.

5. The pharmaceutical composition for use according to anyone of the preceding claims, wherein the D-cysteine derivative is selected from the group comprising D-cystine, homocysteine, meso-lanthionine, S-(2-aminovinyl)-3-methyl-D-cysteine, S-(2-aminovinyl)-D-cysteine and n-acetyl-D-cysteine or a combination of one of more thereof.

6. The pharmaceutical composition for use according to anyone of the preceding claims, wherein said pharmaceutical composition is administered substantially simultaneously or concurrently with one or more additional therapeutic agent or therapy.

7. The pharmaceutical composition for use according to claim 6, wherein the additional therapeutic agent or therapy is selected from the group comprising a D-amino acid oxidase (DAAO) inhibitor, an anticancer agent or therapy, an iron chelator, or a combination of one of more thereof.

8. The pharmaceutical composition for use according to claim 7, wherein the anticancer agent or therapy is selected from the group comprising surgery, radiotherapy, chemotherapy, immunotherapy and hormone therapy, or a combination of one of more thereof.

9. The pharmaceutical composition for use according to claim 8, wherein the DAAO inhibitor is selected from the group comprising a substrate-competitive DAAO inhibitor, a FAD-competitive DAAO inhibitor and a substrate- and FAD-competitive inhibitor, or a combination of one of more thereof.

10. The pharmaceutical composition for use according to claim 9, wherein the DAAO inhibitor is selected from the group comprising 3-substituted 5-hydroxy-1,2,4-triazin-6(1H)-one, benzoate, sodium benzoate, o-aminobenzoate, anthranilate, substituted quinilinones, 4H-furo[3,2-b]pyrrole-5-carboxylic acid, 6-Chloro-1,2-benzisoxazol-3(2H)-one (CBIO), Adenosine diphosphate, Chlorpromazine, and 2-(2,5-dimethylphenyl)-6-fluorobenzo[d]isothiazol-3(2H)-on, 6-hydroxy-3-phenethyl-1,2,4-triazin-5(2H)-one, 3-((6-fluoronaphthalen-2-yl)methylthio) -6-hydroxy-1,2,4-triazin-5(2H)-one, or a derivative or combination of one or more thereof.

11. The pharmaceutical composition for use according to any one of the preceding claims, wherein the D-cysteine isomer is not L-cysteine.

12. A pharmaceutical composition comprising a D-cysteine or a derivative thereof, an isomer or a salt of said D-cysteine or derivative, for use in the treatment and/or prevention of a ferrotoxic disease or an iron related disease or disorder.

13. The pharmaceutical composition of claim 12, wherein the ferrotoxic disease or the iron related disease or disorder is selected from the group comprising hereditary or secondary hemochromatosis, African iron overload, sickle cell disease, thalassemia, X-linked sideroblastic anemia, and very rare protein transport disorders such as aceruloplasminemia or a transferrinemia.

14. A pharmaceutical composition comprising a D-cysteine or a derivative thereof, an isomer or a salt of said D-cysteine or derivative, for use in method of treatment and/or prevention of a cancer in a patient, wherein the method comprises: (i) determining whether the cancer from the patient expresses the cysteine/glutamate antiporter xCT; and (ii) if the cancer from the patient expresses said cysteine/glutamate antiporter xCT, administering to the patient an effective amount of said pharmaceutical composition.
